# EUROPEAN PATENT SPECIFICATION

(11) **EP 4 146 854 B1**
(45) Date of publication and mention of the grant of the patent: **17.09.2025**
(21) Application number: 21730461.7
(22) Date of filing: 05.05.2021
(51) Int. Cl.: D01H 13/22, G01B 11/30, G01N 21/89, G01N 33/36

(54) **A METHOD FOR DETECTING STRUCTURAL DEFECTS OF A FASCIATED YARN, A DEVICE FOR PERFORMING THE METHOD, A COMPUTER PROGRAM FOR THE DEVICE AND A COMPUTER READABLE MEDIUM WITH THE COMPUTER PROGRAM**
VERFAHREN ZUM DETEKTIEREN VON STRUKTURELLEN GARNFEHLERN EINES BÜNDELGARNS, ENTSPRECHENDE VORRICHTUNG, ENTSPRECHENDES COMPUTERPROGRAMM UND ENTSPRECHENDES COMPUTERLESBARES MEDIUM
PROCÉDÉ DE DÉTECTION DE DÉFAUTS STRUCTURELS D'UN FIL FASCIÉ, DISPOSITIF CORRESPONDANT, PROGRAMME D'ORDINATEUR CORRESPONDANT ET SUPPORT LISIBLE PAR ORDINATEUR CORRESPONDANT

(30) Priority: 06.05.2020 EP 20173295
(43) Date of publication of application: 15.03.2023
(73) Proprietor: Rieter CZ s.r.o., 562 01 Ústí nad Orlicí (CZ)
(72) Inventor: KOUSALIK, Pavel, 562 03 Usti nad Orlici (CZ); BERAN, Zdenek, 563 01 Lanskroun (CZ); LUDVICEK, Josef, 562 01 Usti nad Orlici (CZ)
(74) Representative: Musil, Dobroslav
(86) International application number: PCT/CZ2021/050046
(87) International publication number: WO 2021/223784

(56) References cited:
- EP-A1- 2 848 565
- EP-A2- 2 827 132
- CH-A5- 643 060
- DE-A1- 102009 051 426

## Description

### Technical field

The invention relates to a method for detecting structural defects of fasciated yarn comprising a core from parallel fibers which is bound together by wrapper fibers and wild fibers as fibers wound around the yarn core in helices having a variable pitch or perhaps even a variable direction or some of the wild fibers are wound around the yarn core imperfectly.

The invention also relates to a device for detecting structural defects of fasciated yarn.

Furthermore, the invention relates to a computer program (product) containing instructions causing the device for detecting structural defects of fasciated yarn to detect the defects of fasciated yarn during its production, processing or analysis.

In addition, the invention relates to a computer readable medium having this computer program stored therein.

### Background art

Yarn is produced on textile machines which are provided with a suitable device for converting a suitably arranged fiber sliver into the form of yarn. Various methods and associated yarn production devices are known. Yarn is produced, for example, on ring spinning machines, rotor spinning machines, as well as on air-jet spinning machines which have made considerable progress, especially recently.

Various types of yarns are known, the properties and structure of the individual yarns being determined and influenced by the yarn production technology and the settings of the respective production machine.

One of the types of the yarns produced is fasciated yarn which comprises a core which consists of a bundle of substantially parallel fibers bound together by a wrapping layer of fibers. The wrapping layer of fibers compresses the parallel fibers which constitute the yarn core, thereby increasing the friction forces between the individual yarn fibers which are in direct contact. This compression has a positive effect on the overall yarn strength. The wrapping layer of fibers contains wrapper fibers and wild fibers. The wrapper fibers are helically wound around the yarn core in a relatively stable manner in helices with a relatively precise pitch. It is true that the wild fibers are also wound around the yarn core in helices, but these helices have a variable pitch or perhaps even a variable direction or some of the wild fibers are wound around the yarn core imperfectly. This imperfect wrapping of the wild fibers around the fasciated yarn core manifests itself in the overall yarn structure basically in two ways. The first manifestation of imperfect wrapping of the wild fibers around the fasciated yarn core is the slackening of such a wild fiber between the two of its ends which are wrapped around the fasciated yarn core, i.e., the middle part of such a wild fiber forms a loop or tuft on the fasciated yarn surface. The second manifestation of imperfect wrapping of the wild fibers around the fasciated yarn core is the incomplete wrapping of the end of a wild fiber around the yarn core, where this unwound end of the wild fiber protrudes from the yarn core, and, consequently, from the yarn itself. In the overall structure of fasciated yarn, the ends of the wrapper fibers and in some cases also the ends of the core fibers, i.e., the ends of the parallel fibers constituting the core of the yarn, protrude from the fasciated yarn core. A typical production technology in which fasciated yarn is formed is production of yarn on air-jet spinning machines, which is very efficient and economically profitable. Fasciated yarn formed on jet spinning machines is also referred to as "air-jet yarn" and is characterized by the above-described specific structure comprising a core from parallel fibers which is wrapped by wrapper fibers and wild fibers, i.e., the core is wrapped by a wrapping layer of fibers.

Nowadays, for yarns produced by various technologies, i.e., not only for the above-described fasciated yarn, one of the indicators of yarn quality evaluation is the indicator of yarn hairiness. The yarn hairiness is characterized by a plurality of fiber ends or fiber loops which protrude from the yarn or from a flat textile (fabric, jersey fabric, warp knit fabric, fleece) or which are freely movable. The criterion for the assessment of the yarn hairiness is thus essentially the number of fibers protruding in the direction perpendicular to the yarn or a measured distance of the fiber ends from the yarn.

The technology of producing fasciated yarn, e.g. on air-jet spinning machines, also brings new problems and technological challenges that were unknown to the yarn production technologies used so far, i.e., ring and rotor spinning. One of these relatively recent problems associated with fasciated yarn is accidental occurrence of places or length sections in the fasciated yarn which exhibit specific structural defects of fasciated yarn, whereby these specific structural defects of fasciated yarn are not noticeable in the fasciated yarn at first sight, but they are objectively manifested, e.g., by insufficient strength of fasciated yarn in that particular place or section and/or by unsatisfactory appearance of fasciated yarn in that particular place or section, whereby the manifestations of such structural defects are mainly detected only during the subsequent processing of the fasciated yarn already produced, e.g., during the finishing of fasciated yarns or during the production of textile products from fasciated yarns (weaving, knitting). In some cases, these structural defects of fasciated yarns show up only in the completed product, either before dyeing or after dyeing, e.g., on the finished fabric or knitwear, where these defects, previously invisible to the human eye, become differences of certain places of the fabric or knitwear, etc., which are to the eye visible. Hereinafter, these defects will be called "structural defects" because their origin is in the altered structure of a certain section of fasciated yarn.

These structural defects of yarn are currently not detectable by conventional methods of online fasciated yarn quality control, e.g., in the production of yarn on air-jet spinning machines, and, moreover, they are not detectable at all by using non-contact methods of monitoring the quality of fasciated yarn during production of fasciated yarn. Generally, the users of air-jet spinning technology, i.e., producers of fasciated yarns, try to prevent occurrence of these structural defects by empirical methods by means of setting the parameters of the production process of fasciated yarns whereby usually, it is impossible to check whether the setting was successful already during yarn production, but this can be checked in feedback after the yarn is produced, e.g., by detailed yarn examination in the laboratory or by receiving a response from the subsequent yarn processor, which is highly undesirable due to high costs of laboratory tests of a large quantity of fasciated yarn or due to the high risk of producing a potentially large quantity of fasciated yarn of insufficient quality delivered to the customer.

The process of yarn formation and the influence of its specific structure on air-jet spinning machines is thoroughly described, e.g., in the doctoral thesis by the author Moaaz Ahmed Samy Moustafa Eldeeb, entitled Different Approaches for Predicting Air Jet Spun Yarn Strength, 2017, available at https://dspace.tul.cz/handle/15240/26033. The thesis aims to describe the process of yarn formation, to investigate the influence of selected technological parameters of an air-jet spinning machine on yarn properties, especially on the strength of air-jet yarn and, most of all, to shed light on the problem of the prediction of air-jet yarn strength. The thesis deals with an analysis of air-jet yarn structure and provides calculation of the strength of a yarn core as a bundle of parallel fibers, as well as calculation of the strength of rapping layer of fibers as a bundle of fibers wrapped around the yarn core in the form of a helix. The thesis also considers the interaction effect between the fibers in the wrapping of the yarn and the fibers in the yarn core. In addition to fiber parameters, also yarn structural parameters are used as input parameters of a model for the calculation of the theoretical yarn tenacity at short gauge length. As an alternative approach to predict yarn strength (on short length sections), a statistical model has been presented. By using this model, the influence of the tensile tester gauge length on the strength of ring, rotor, and air jet spun yarn and its coefficient of variation has been investigated.

One of the structural defects of fasciated yarn, e.g., the yarn produced on an air-jet spinning machine, is occurrence of places or sections of yarn with reduced strength of fasciated yarn, but at the same time without significantly affecting the mass and/or diametric characteristics of fasciated yarn or without significantly affecting the parameter of yarn hairiness in these places or sections of fasciated yarn. Nevertheless, it is the monitoring of the mass and/or diametric characteristics of fasciated yarn and the monitoring of yarn hairiness that is used for the evaluation of fasciated yarn quality during the production of fasciated yarn, e.g., on air-jet spinning machines. The above-mentioned hairiness of fasciated yarn is evaluated essentially as a statistical number obtained by measuring long sections of yarn, i.e., sections of yarn having tens to hundreds of meters in length, whereby the hairiness of fasciated yarn is afterwards recalculated back to 1 cm or 1 m of fasciated yarn length, which is not suitable for the assessment of yarn defects occurring only locally or only on short sections of fasciated yarn.

For detecting occurrence of places or sections of fasciated yarn with reduced yarn strength, but at the same time without significantly affecting the mass and/or diametric characteristics of fasciated yarn or without significantly affecting the parameter of yarn hairiness in these places or sections of fasciated yarn on an air-jet spinning machine, the solution disclosed in EP 2 565 307 can be used. This document describes a sensor for detecting changes in tension in moving yarn on an air-jet spinning machine, wherein this sensor comprises a contact element in the form of a pulley through which the measured yarn passes, whereby the pulley is mounted on an arm of a deformation measuring member and the changes in yarn tension caused by local occurrence of a structural yarn defect result in changes in the deformation of the deformation measuring member. These changes in the deformation of the deformation measuring member are detected, e.g., by strain gauges, and evaluated to determine occurrence of places or sections of fasciated yarn with reduced yarn strength, the so-called weak yarn.

Additional mechanical action on the fasciated yarn being produced (friction and increased yarn loading) in the solution according to EP 2 565 307 may cause reduction in the quality of fasciated yarn being produced, disrupt the continuity of fasciated yarn production, reduce the productivity of fasciated yarn production or even affect the subsequent processing of fasciated yarn. The natural occurrence of impurities in textile manufacturing processes can cause mechanical problems of the measuring member itself. As it is a mechanical or mechatronic device, it is also relatively expensive and demanding in terms of service, adjustment for different types and different fineness of yarns produced and in terms of long-term stability of the yarn production process, etc.

Many types of yarn sensors for yarn monitoring are generally known. Known are contact yarn sensors, see the above-mentioned patent EP 2 565 307, as well as non-contact yarn sensors.

Contact yarn sensors, i.e., sensors which, in order to determine yarn parameters, require the yarn to be in direct contact with the measuring member of the sensor during the measurement, are mainly used in laboratories and for additional yarn quality evaluation, because during the measurement, yarn must either be at rest or move only at a limited speed. However, as EP 2 565 307 proves, also contact yarn sensors are used in the production of yarn on air-jet spinning machines, especially if there is no sufficiently reliable and powerful non-contact alternative to the yarn sensor.

Non-contact yarn sensors are based on various physical principles, such as capacitive sensors, ultrasonic sensors, electric charge sensors, or the non-contact yarn sensors are based on optical principles.

Different types of optical yarn sensors are described in numerous documents, e.g., CZ 306117, CZ 305265, EP 2827132, US371274, EP 0 627 623, JP 4 756 411, US 6 219 135, US 5 270 787, US 5 654 554, US 5 521 395, WO 2011 026 249 and others.

A common disadvantage of the background art in the field of optical yarn sensors is the inability to monitor and evaluate occurrence of the above-described or mentioned structural defects manifested mainly only in the subsequent processing of yarns. Neither the methods and techniques used so far for non-contact evaluation of fasciated yarn quality and quantitative (mass or diametric) parameters used so far for non-contact evaluation of fasciated yarn quality, e.g., during its production on an air-jet spinning machine, such as CV, IPI, yarn diameter, yarn fineness, which are basically based on monitoring mass and/or diametric characteristics and defects of fasciated yarn, nor techniques of evaluating the fasciated yarn hairiness during its passage through the places or sections of fasciated yarn with structural defects, substantially exhibit any indication that there is either an increased likelihood of occurrence of a specific structural defect of fasciated yarn in that specific place or section of the produced fasciated yarn or that a structural defect of fasciated yarn actually occurs in that place.

The object of the invention is therefore to eliminate or at least minimize the disadvantages of the background art, especially to provide a method, a device, software and a software carrier for non-contact measurement of occurrence of structural defects of fasciated yarn, i.e., yarn comprising a core from parallel fibers, whereby the core is wrapped by a wrapping layer of fibers which comprises wrapper fibers and wild fibers.

### Principle of the invention

The object of the invention is achieved by a method for detecting structural defects of fasciated yarn, comprising a core formed by parallel fibers, whereby the core is bound together by a wrapping layer of fibers which comprises wrapper fibers and wild fibers as fibers wound around the yarn core in helices having a variable pitch or perhaps even a variable direction or some of the wild fibers are wound around the yarn core imperfectly, in which an image of moving yarn is captured by at least one optical sensor on at least a portion of the yarn length, whereby the optical yarn sensor comprises at least one row of radiation-sensitive elements and at least one radiation source, whereby the captured image of the yarn is processed by a digital image analysis method to obtain data on yarn surface structure and from the obtained data, the yarn surface structure along the yarn length is evaluated, the changes in the yarn surface structure are compared with preset criteria, and if the changes in the yarn surface structure exceed these pre-set criteria, the relevant place of yarn is determined as a place of occurrence of a structural defect of yarn, where the structural defects of fasciated yarn are an insufficient strength of fasciated yarn in particular place or section and/or unsatisfactory appearance of fasciated yarn in particular place or section, all without significantly affecting the mass and/or diametric characteristics of fasciated yarn or without significantly affecting the parameter of yarn hairiness in these places or sections of fasciated yarn).

In order to simplify and speed up the method for capturing an image of fasciated yarn and for the image analysis of this image of fasciated yarn for the evaluation of occurrence of structural defects of fasciated yarn, it is advantageous if the yarn image is captured by the optical yarn sensor along the yarn edges, the captured yarn image is processed by a digital image analysis method to obtain data on the yarn structure in the area along the yarn edges, from the data obtained, changes in the yarn structure along the yarn edges are evaluated, these changes in the yarn structure along the yarn edges are compared with the pre-set criteria and if these changes in the yarn structure along the yarn edges exceed the pre-set criteria, the relevant place of yarn is classified as a place of occurrence of a structural defect of yarn.

It is advantageous for capturing an image of fasciated yarn if the optical yarn sensor captures an image of a yarn envelope in the area along the yarn edges, which is a limited area of the image field along the yarn edges that extends both into the yarn and into the zone outside the yarn where this zone is situated along the yarn edges. At the same time, an analysis of lateral deviations of the image of the yarn envelope from the straight direction of yarn is made by a digital image analysis method and/or an analysis of disorder of the yarn structure is performed.

According to one embodiment, to analyze the lateral deviations of the image of the fasciated yarn envelope from the straight direction of yarn, changes in the positions of the edges of the yarn image relative to the optical yarn sensor are evaluated, wherein at least one range of frequencies of the spectrum of the positions of the yarn edges is determined or set and the spectrum of the positions of the edges of the yarn image in the at least one determined or set range of frequencies is monitored and continuously evaluated. From the monitored and continuously evaluated spectrum of the positions of the edges of the yarn image in the at least one determined or set range of frequencies, waviness of the yarn surface is determined as a function of the deviations of the yarn envelope from the straight direction of yarn. Current lateral deviations of the image of the yarn envelope from the straight direction of yarn are continuously statistically evaluated and compared with a reference value of the waviness of the yarn surface, wherein the reference value of the waviness of the yarn surface is formed by the long-term average of the values of the waviness of the yarn surface from the same spinning unit and/or by the long-term average of values of the waviness of the yarn surface from multiple spinning units set to produce the same yarn, and/or the reference value of the waviness of the yarn surface is set according to the current production or processing or measuring parameters at a spinning station of an air-jet spinning machine, e.g., by means of a central control system of the production or processing or measuring machine. A structural defect of yarn is detected upon occurrence of the current deviations of the waviness of the yarn surface with respect to the reference value of the surface waviness by more than is the set decision threshold.

According to one embodiment, the above-mentioned range of the analyzed frequencies of the spectrum of the positions of the edges of fasciated yarn is set depending on the current yarn parameters, especially on the type of fibrous material for yarn production, the speed of yarn movement, the spinning pressure in the spinning nozzle, the fineness of the spun yarn and depending on the current components of the spinning unit, in particular on the type of spinning nozzle.

According to one embodiment, for analyzing disorder of the fasciated yarn structure, at least one area for monitoring an image of wild fibers is determined or set on at least a portion of the yarn length significant for monitoring disorder in the yarn structure, and in this area the yarn disorder is monitored and evaluated. From the disorder in the yarn structure, yarn wildness is determined as a function of occurrence of irregularities in the structure of fasciated yarn and/or as a function of occurrence of irregularities in the surface structure of fasciated yarn. The yarn wildness is monitored and compared with a reference value of the yarn wildness, whereby the reference value of the yarn wildness is either the long-term average of the values of the yarn wildness from the same spinning unit and/or it is the long-term average of the values of the yarn wildness from multiple spinning units set to produce the same yarn and/or the reference value of the yarn wildness is set according to current production or processing or measuring parameters of the production or processing or measuring machine and a structural defect of the yarn is detected upon occurrence of deviations of the current yarn wildness from the reference yarn wildness by more than the set decision threshold.

It is advantageous if the area for monitoring the image of wild fibers on at least a portion of fasciated yarn length is set depending on the current yarn parameters, especially on the type of fibrous material for the yarn production, the speed of yarn movement, the spinning pressure in the spinning nozzle, the fineness of the spun yarn and depending on the current components of the spinning unit, especially the type of spinning nozzle.

To increase the accuracy and reliability of detecting a structural defect of fasciated yarn, the analysis of the lateral deviations of the image of the yarn envelope from the straight direction of yarn and the analysis of the disorder in the yarn structure are performed concurrently.

For the complex determination of the fasciated yarn parameters simultaneously with the capturing of the yarn image for detecting a structural defect of yarn, the optical sensor of yarn quality senses at least one yarn diameter parameter, in particular the yarn diameter.

The principle of the device for detecting structural defects of fasciated yarn, where the device comprises at least one optical yarn sensor with at least one row of radiation-sensitive elements and further comprises at least one radiation source, consists in that the device has means adapted to perform the method for detecting structural defects of fasciated yarn according to the above-mentioned method.

From the point of view of the efficient use of the working means of the production or processing or measuring machine, it is advantageous if the optical yarn sensor is formed by an optical sensor of yarn quality.

To ensure the computational and control performance of the device, it is advantageous if the means adapted to perform the method for detecting structural defects of fasciated yarn according to the above-mentioned method comprise a microprocessor with a memory or a gate array or a customer electronic circuit of the ASIC type, or comprise a combination of at least two of these elements.

The principle of the computer program (product) consists in that it contains instructions causing the device according to the preceding paragraphs to perform the steps of the method for detecting structural defects of fasciated yarn according to the above paragraphs.

The principle of the computer readable medium for the present invention consists in that it has a computer program stored therein, which contains instructions causing the device according to the preceding paragraphs to perform the steps of the method for detecting structural defects of fasciated yarn device according to the above paragraphs.

The invention is based on the fact that occurrence of structural defects of fasciated yarn, i.e., yarn which comprises a core formed by parallel fibers, whereby the core is bound together by a wrapping layer of fibers consisting of wrapper fibers and wild fibers, can be also detected by an optical yarn sensor by using a special method for the detailed processing of obtained data describing the visual character or changes in the visual character of the structure of fasciated yarn, especially the surface structure of fasciated yarn, whereby from the changes in the character of the yarn structure or of the yarn surface structure captured by the optical sensor, it is possible to determine such changes in the yarn structure, or, more specifically, such changes in the yarn surface structure that, on the basis of these changes, it is possible to determine a place of fasciated yarn or a section of fasciated yarn which falls into the category of places or sections on which, in the subsequent yarn processing processes following the yarn production itself, i.e., in the processing phase of the use of the yarn, are manifested structural yarn defects, the so-called processing yarn defects.

Thus, the method according to this invention does not to detect yarn defects which affect yarn quality described by conventional yarn quality parameters, such as occurrence of thin and thick places, hairiness, etc., but it detects faults which, due to their specific character or manifestation, appear on short sections of fasciated yarn only during the subsequent processing of the yarn, e.g. during the production of woven r knitted fabric or after the production of woven or knitted fabric, when these previously hidden defects show up in certain places or areas on this woven or knitted fabric as optical differences of certain places or areas, which are, e.g., based on different colors of these previously indistinguishable places or sections of the yarn after dyeing or due to different light reflectance from these previously indistinguishable places or sections of the yarn, etc. It should be noted that such places or sections of yarn with structural defects do not exhibit the typically monitored mass defects or yarn diameter defects or defects of yarn hairiness, whereby generally these places with structural yarn defects have reduced axial strength, but even this reduced axial strength is still sufficient for the yarn production and for the subsequent processing of such yarns. At the same time, however, it should be noted that if the wrapping layer of fibers is not stable, uniform, etc., the yarn core is not sufficiently compressed. As a result, radial strength of the yarn is reduced, which in turn leads to faster wear of this yarn as a manifestation of a structural yarn defect. Radial strength of yarn is evaluated, for example, by a rolling test. Naturally, optical yarn sensors cannot monitor yarn strength during the production or processing or analysis of fasciated yarn, etc., or they cannot monitor changes in yarn tension during the production, processing or analysis of fasciated yarn.

The solution according to the invention therefore works with the fact that occurrence of places or sections of yarn with structural yarn defects is manifested by a local and brief change in the structure and character of fasciated yarn, which, in the subsequent processing of yarn into a product (fabric) during which the sections mentioned above are mixed with the yarn without structural defects, leads to the formation of a visible difference in the product. It should be noted that this visible difference is not caused by the differences in yarn strength, but it is caused by the differences in the structure and character of fasciated yarn in the place of a structural defect of fasciated yarn.

It should be added that in principle, the optical monitoring and evaluation of places or sections of yarn with structural defects according to the present invention does not restrict other monitoring processes and evaluation of yarn quality performed by optical or other methods, such as monitoring yarn quality from the point of view of occurrence of mass or diameter defects in yarn or from the point of view of yarn hairiness, etc. On the contrary, it can be said that monitoring yarn and evaluating occurrence of places or sections of yarn with structural defects by an optical method, which is the subject-matter of the invention, takes the evaluation of the quality of fasciated yarn to a whole new level, where the evaluation of fasciated yarn is considerably more detailed, because it focuses on occurrence of rapid changes in the yarn structure or rapid changes in the structure of the yarn surface, which is currently not applied in any way during the yarn production or in the subsequent processing or analysis of fasciated yarn.

The subject of a detailed analysis of the character of fasciated yarn according to the present invention is therefore rapid changes in the structure of fasciated yarn, in particular rapid changes in the surface structure of fasciated yarn, especially changes in the wrapping of the yarn core by the wrapper fibers and/or by the wild fibers and occurrence of slackened portions or free ends of the wild fibers, free ends of the other yarn-forming fibers and possible slackening of the yarn core as a result of insufficient wrapping, either directly on the surface of fasciated yarn and/or in a very limited area outside the surface (above the surface) of fasciated yarn. For the purposes of this invention, these rapid changes in the surface structure of fasciated yarn are well characterized by changes in the waviness of the yarn surface and/or by changes in the wildness (disorder) of the yarn surface, according to which such places or sections of fasciated yarn can be classified as places or sections with a structural defect of fasciated yarn.

As follows from the essence of the matter, optical yarn sensors are in principle well-known, especially optical sensors of yarn quality evaluating yarn diameter defects and, if appropriate, also yarn hairiness. These sensors can be used for this invention provided they allow a sufficiently detailed and fast acquisition of a yarn image and the processing of the acquired image data on the visual character of the fasciated yarn structure, or, to put it more specifically, on the visual character the surface structure of fasciated yarn. Preferably, sensors whose sensing element or elements provide sufficient resolution for capturing a detailed yarn image for evaluating the surface structure of fasciated yarn are used. Such a sensing element (or elements) is, for example, a single-row or even a multi-row optical sensing element with a sufficient density of radiation-sensitive elements arranged in a row next to one another transversely to the path of yarn movement, e.g., at a spinning station of an air-jet spinning machine or at a workstation of a winding machine, etc. Another advantage of the solution according to the invention is the fact that the image data on the visual character of the fasciated yarn structure, or image data on the visual character of the surface structure of fasciated yarn, are obtained by the optical yarn sensor without any additional mechanical components acting on the fasciated yarn, which is therefore not subjected to any additional stress, which makes it possible to maintain high speeds of the production, processing or measuring of the fasciated yarn and not to affect the quality of the produced, processed or measured fasciated yarn. In addition, any changes and adjustments to the settings for the optical monitoring and evaluation of structural yarn defects in different types of fasciated yarn can be performed merely by software modifications and changes of the relevant parameters of the evaluation algorithm within the device according to the invention, which is fast and relatively inexpensive. Another advantage is that the optical yarn sensor can be common both for sensing fasciated yarn according to the present invention and for hitherto used methods of optical sensing and evaluation of yarn quality that are based on the principle of diameter yarn defects, yarn hairiness, etc., which is economically advantageous and simplifies the construction of a workstation, e.g., of a spinning unit of an air-jet spinning machine or a workstation of a winding machine. At the same time, however, it should be noted that the present invention can also be implemented as a solution independent of the existing means of evaluating fasciated yarn quality.

From the point of view of fasciated yarn image processing, digital image analysis is used, for example a specific image analysis procedure which is here based on, but not limited to, this specific procedure of image analysis, on the representation of a signal in the frequency domain, which is an expression of the output signal of the optical yarn image sensor in which this signal is understood as the sum of a series of suitably selected periodic functions, which is a different approach from the standard approach in which the output signal of the optical yarn image sensor is understood as a function of time. In practice, the use of trigonometric functions, i.e., the sine and cosine functions, has proved to be the most effective. To do this, e.g., by the Fourier transform, the given output signal is decomposed into harmonic components (1., 2., 3., ...) and the frequency spectrum of the given signal is created. The output of this processing are complex numbers, because each frequency has its own amplitude and phase. In this case, amplitude and phase spectra are usually used. The information about the given signal is carried by both spectra together, one without the other does not contain complete information. The frequency spectrum is a representation of certain signal frequencies in terms of their repetition, frequency strength or also amplitude. The amplitude spectrum is the dependence of the amplitude of the measured quantity on the frequency. From the frequency spectrum, the frequencies of simple oscillating motions that have the greatest influence on the resulting complex course are visible. In our case, it is also possible to use the so-called power spectrum, in layman's terms, "what power the frequency component of the signal has", which can be simplified as the square of the amplitude spectrum, but it is necessary to take into account the fact that the power spectrum indicates energy signal ratios and to a certain extent this is an important difference from the classical approach using the frequency domain.

In our case, it is also possible to determine, e.g., according to the production parameters of the spinning machine, the winding machine, etc., such as the spinning speed, the speed of fasciated yarn movement, etc., a range of frequencies of the frequency spectrum which are important for the purposes of the present invention and it is possible to determine the "energy" of this range of the power spectrum. It is then possible to work with the value thus determined, for example, for determining the criteria of the waviness of the yarn surface.

Another option is using a digital filter for signal processing. In this case, the input signal is processed by a digital bandpass filter, through which only the signal of specific frequencies can pass to the filter output, whereby the specific frequencies are set according to the machine parameters or are determined in a suitable manner.

For the purposes of the invention, the waviness of the yarn surface means the deviations of the yarn envelope from the straight direction of yarn. The waviness of the yarn surface, or the yarn waviness, is preferably determined by storing the positions of the first, or possibly also of the last, continuously shaded radiation-sensitive element of the optical sensing element from each image of the yarn on the linear optical sensing element, thereby detecting the current position of the yarn edge and thus monitoring the yarn envelope which is further analyzed by the method according to the invention. Furthermore, for each yarn image on the linear optical sensing element, at first the fibers protruding from the yarn core are filtered off and then the position of the shaded radiation-sensitive element of the optical sensor of at least one edge of the yarn core is determined. The position of the yarn edge is stored and the envelope of the yarn core is monitored. Subsequently, the envelope of the yarn core is analyzed according to the method of the invention.

Under the term "wildness of fasciated yarn" is basically understood a new qualitative parameter of the evaluation of fasciated yarn which is different from the qualitative parameters used today for the evaluation of fasciated yarn, whereby it is also different from the parameter called yarn hairiness. In principle, the yarn wildness is an indicator (characteristic) of fasciated yarn which describes and quantifies occurrence of irregularities in the structure of fasciated yarn, or, more specifically, irregularities in the surface structure of fasciated yarn, which are mainly due to the irregularities in the wrapping of the yarn core by the wrapping layer of fibers and other irregularities in the yarn structure as such. The yarn wildness thus takes into account both the wild fibers twisted on the yarn core, their number, direction of the fibers slackened on the yarn core, and also takes into account occurrence of untwisted free ends or other parts of the wild fibers. Moreover, the parameter of the yarn wildness takes into account occurrence of the slackening of the yarn core due to insufficient local wrapping of the yarn core. Moreover, the parameter of yarn wildness takes into account occurrence of fibers protruding from the yarn core and passing through a limited area along the edges of the yarn core into the yarn hairiness evaluation area, etc. The wildness of fasciated yarn is thus a characteristic describing the degree of the disorder in the yarn structure or the degree of the disorder in the yarn structure on short length sections of fasciated yarn.

One approach to monitoring and recognizing the structure of fasciated yarn is monitoring and recognizing untwisted free ends of the wild fibers in a narrow band along the edges of the yarn core, when a yarn image is captured by the optical yarn sensor, whether it is the yarn image obtained by the yarn casting its shadow on at least one row of radiation-sensitive elements of the optical sensing element or whether it is the yarn image obtained by the reflection of the radiation from the yarn and the incidence of the reflected radiation on at least one row of radiation-sensitive elements of the optical sensing element. From the captured yarn image, the position and extent (size, thickness, diameter) of the yarn core are determined, and all the objects which are recorded in the set narrow band of the image along the thus identified yarn core are considered objects that fall within the evaluation of the wildness of fasciated yarn.

Another approach to monitoring and recognizing the structure of fasciated yarn is to use image analysis of the yarn image with recognition of the position and extent (size, thickness, diameter) of the yarn core, of the untwisted free ends of the wild fibers, as well as of the wild fibers wrapped around the yarn core, of the free fiber ends of the wrapper fibers, of the free ends of the fibers protruding from the yarn core, etc., i.e., of the irregularities situated on the yarn surface.

Subsequently, e.g., the number of activated (shaded or irradiated by the radiation reflected from the yarn) radiation-sensitive elements of the sensing element is added up, whereby the radiation-sensitive elements are situated outside the identified image of the yarn core in the set narrow band along the identified yarn core. Optionally, these irregularities are monitored in the set narrow band along the yarn core within the yarn image captured (e.g., even across several rows of the sensitive elements of the sensor), including image analysis of the overall yarn image, which enables to recognize irregularities in the structure of fasciated yarn along the entire width of the captured image of fasciated yarn, etc.

As already mentioned above, the image data on the fasciated yarn, or, more specifically, the image data on the surface structure of fasciated yarn, can also be obtained by methods of image analysis of a yarn image, from which the data describing the visual character of fasciated yarn or, more specifically, the data describing the visual character of the surface structure of fasciated yarn, are determined by image analysis methods and these data are used by the method according to the invention to detect places or sections of fasciated yarn with structural yarn defects. The image analysis is possible, e.g., with the aid of convolutional methods, filtering methods, detection of edges or objects in the image, etc., e.g., according to the publication by the authors Klima, M., Bernas, M., Hozman, J., Dvořák, P.: Zpracování obrazové informace (Image information processing), textbook of CVUT, Praha 1996, then according to the publication An Introduction to Digital Image Processing, Frédéric Patin aka YOV408, available at http://teachme.free.fr/ImageProc.pdf, and according to the publication by the authors O. Zmeškal, O. Sedlák, M. Nežádal: Metody obrazové analýzy dat (Methods of image data analysis) (5/2002) and others.

### Description of drawings

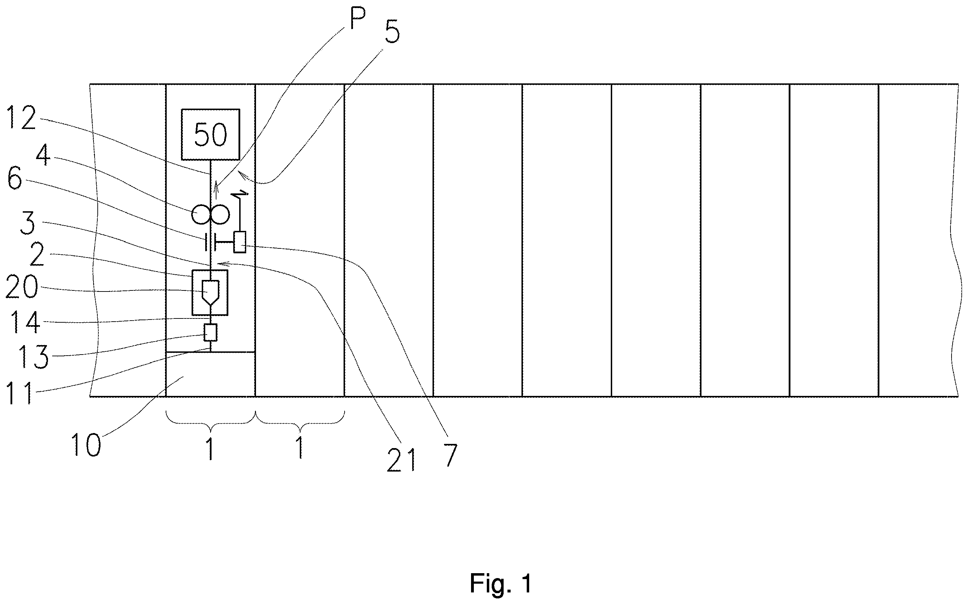

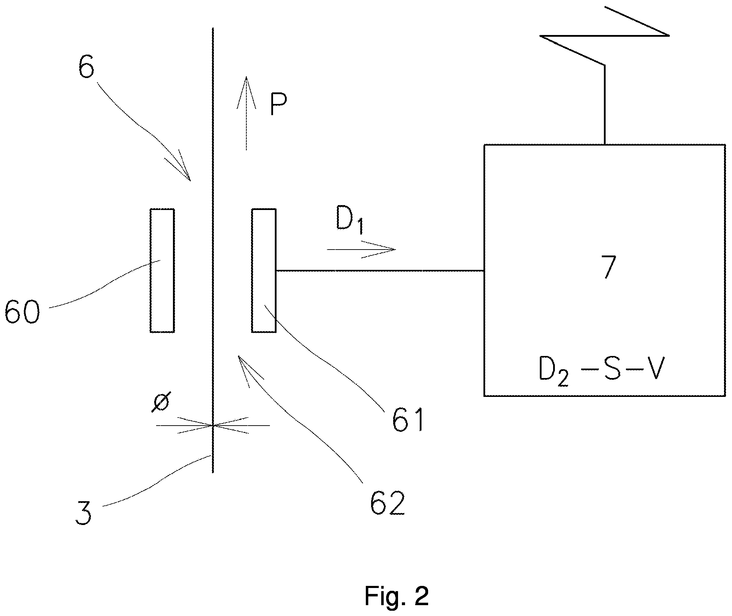

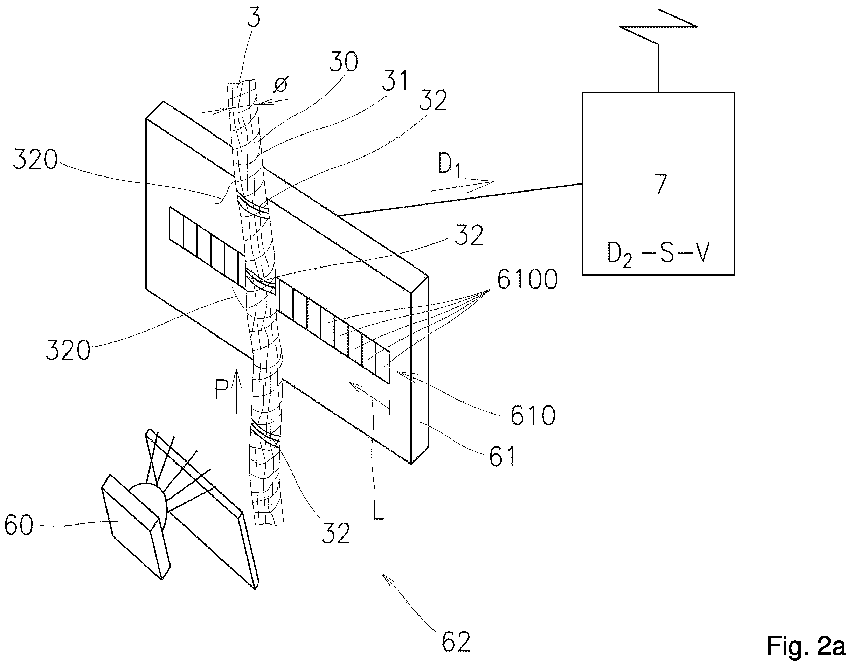

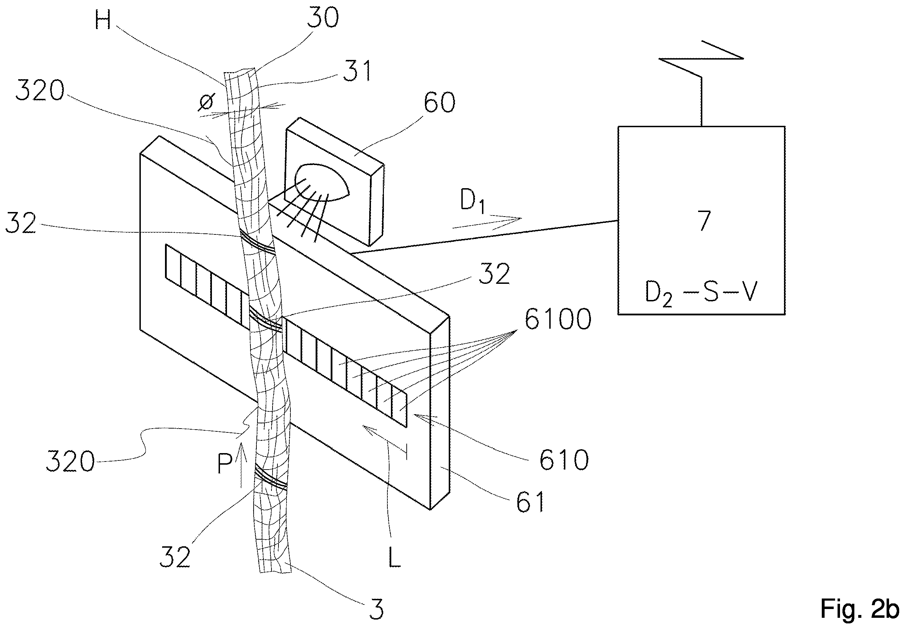

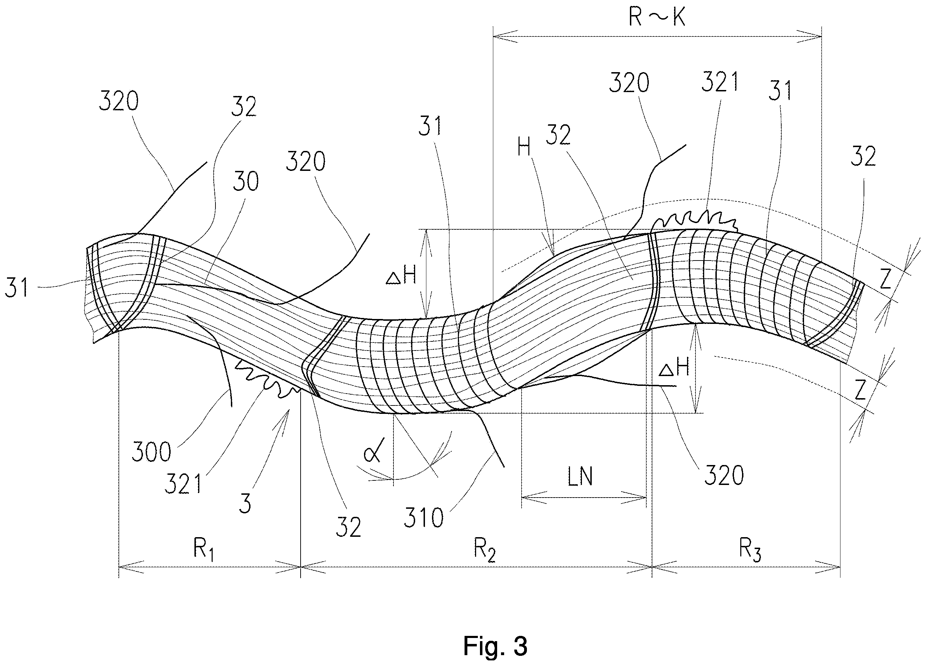

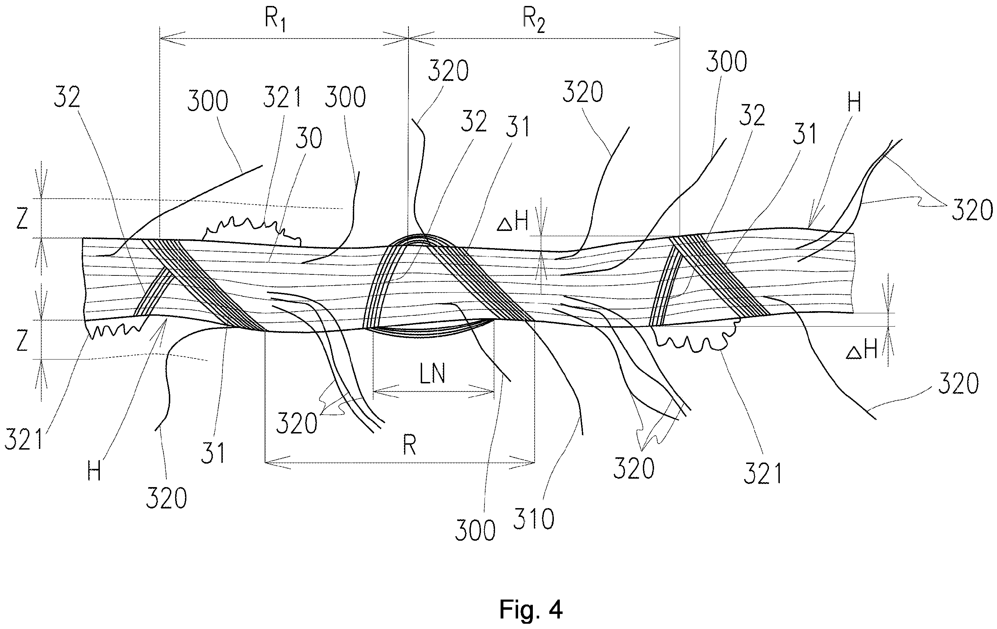

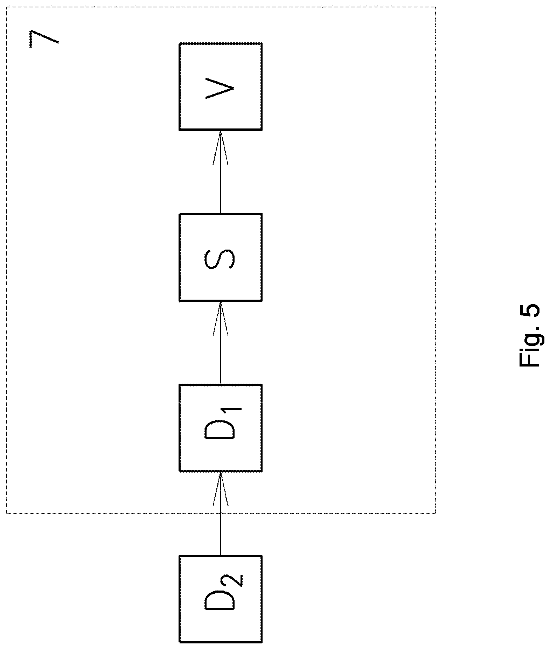

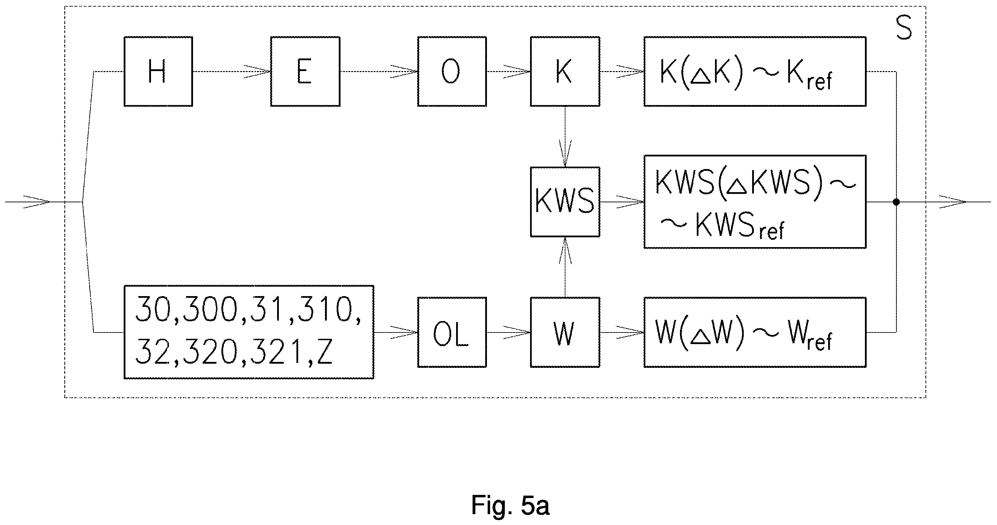

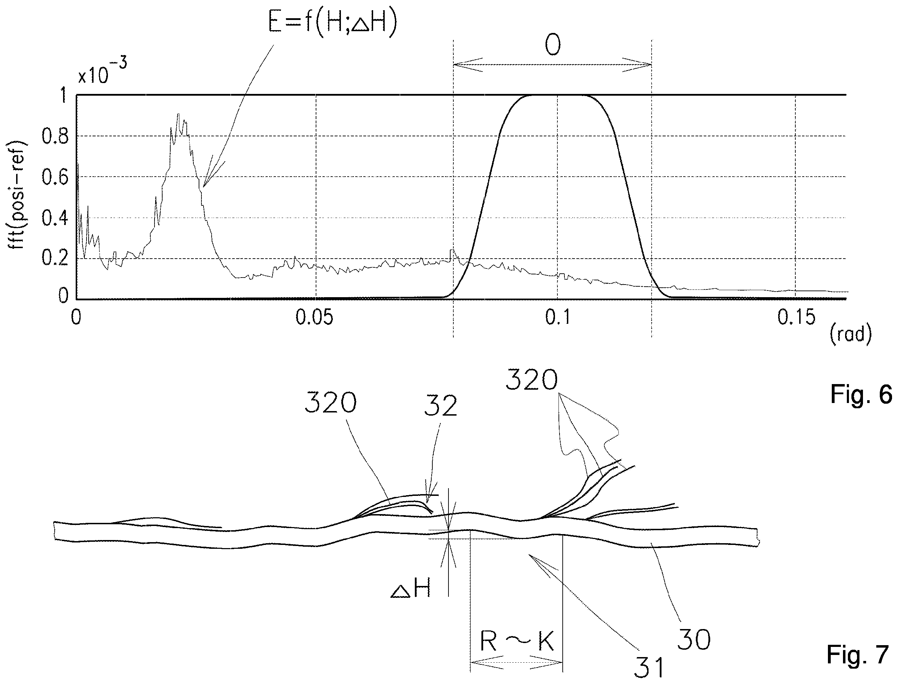

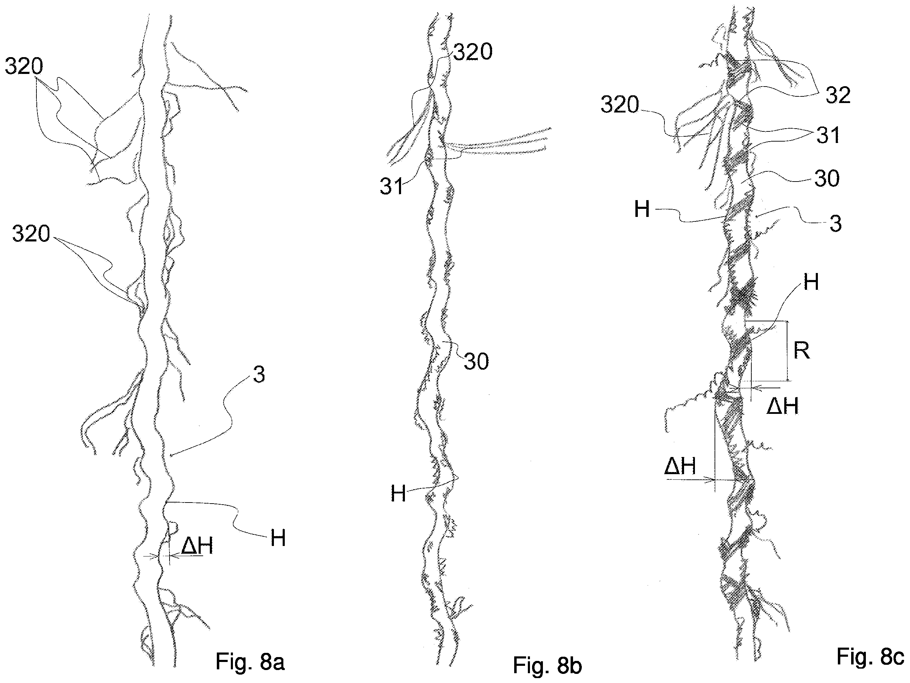

The invention is schematically represented in the drawings, wherein Fig. 1 shows an arrangement of an air-jet spinning machine and one of its spinning stations, Fig. 2 shows a detail of a general arrangement of a device according to the invention, Fig. 2a shows a detail of an arrangement of one embodiment of the device according to the invention, Fig. 2b shows a detail of an arrangement of a second embodiment of the device according to the invention, Fig. 3 shows an example of the structure of fasciated yarn and its parts according to the invention, Fig. 4 shows an example of the structure of fasciated yarn with structural defects, Fig. 5 shows a rough flow chart of the evaluation of structural defects of fasciated yarn according to the invention, Fig. 5a shows a detailed flow chart of the evaluation of structural defects of fasciated yarn according to the invention, Fig. 6 shows the spectrum of the position of the edges (changes in the position of the edges) of the yarn surface in the frequency domain with one determined or set range of frequencies which are significant for monitoring the spectrum of the position of the edges of the yarn surface (changes in the position of the edges), Fig. 7 shows a captured image of the actual fasciated yarn subjected to the detection of structural defects according to the invention, Fig. 8a shows an image from the detection of the edges of the actual fasciated yarn for the purposes of this invention, captured, e.g., by the arrangement of the sensor of Fig. 2a, Fig. 8b shows an image from the detection of the yarn core, the wrapper fibers and the wild fibers of the actual fasciated yarn for the purposes of the invention, captured, e.g., by the arrangement of the sensor of Fig. 2b, Fig. 8c shows an image captured from the detection of the yarn structure for the purposes of this invention created by a combination of the captured images of Fig. 8a and Fig. 8b.

### Examples of embodiment

The invention will be described with reference to an exemplary embodiment of a method for detecting structural defects of fasciated yarn 3 during the production of fasciated yarn 3 at a spinning station 1 of an air-jet spinning machine. Nevertheless, the invention is applicable in numerous other textile machines, in particular winding machines and machines for measuring the quality of fasciated yarn 3. The invention is thus applicable not only for online non-contact detection of structural defects of fasciated yarn 3 during the production of fasciated yarn 3, but also in phases of the subsequent processing of fasciated yarn 3, e.g., when winding the fasciated yarn 3, when processing the fasciated yarn 3 into a subsequent product, etc.

When only the term "yarn" is used in the following text, it is to denote "fasciated yarn 3", which comprises a core 30 formed by parallel fibers, the core 30 being bound together with a wrapping layer of fibers which comprises wrapper fibers 31 and wild fibers 32.

The method for detecting structural defects of fasciated yarn 3, which comprises a core 30 formed by parallel fibers, the core 30 being bound together with a wrapping layer of fibers which comprises wrapper fibers 31 and wild fibers 32, uses the fact that an image of yarn 3 is captured by at least one optical sensor 6 of yarn 3 on at least part of the yarn 3 length. The optical yarn sensor 6 comprises at least one row 610 of radiation-sensitive elements 6100 and at least one radiation source 62 and the optical sensor 6 is connected to electronics 7 equipped with software. The captured image of yarn 3 is processed by a digital image analysis method to obtain data on the yarn 3 surface structure and from these obtained data, changes in the structure of the yarn 3 surface along the yarn 3 length are evaluated. These changes in the structure of the yarn 3 surface are compared with pre-set criteria and if these changes in the structure of the yarn 3 surface exceed the pre-set criteria, the relevant place of yarn is determined as the place of occurrence of a structural defect V of fasciated yarn 3.

Preferably, the optical sensor 6 of yarn captures an image of fasciated yarn 3 along the edges H of yarn 3 and the captured image of yarn 3 is processed by the digital image analysis method to obtain data on the yarn 3 structure along the edges of the yarn and from the data obtained, changes in the yarn structure along the edges of the yarn 3 are evaluated. These changes in the yarn structure along the edges of the yarn 3 are compared with the pre-set criteria and if these changes in the structure of the yarn 3 surface along the edges of yarn exceed the pre-set criteria, the relevant place of yarn is determined as the place of occurrence of a structural defect V of fasciated yarn 3.

It is advantageous if the optical sensor 6 of yarn captures an image of the yarn envelope along the edges of the yarn 3 and analysis of lateral deviations of the image of the envelope of the yarn 3 from the straight direction of yarn is made by the digital image analysis and/or analysis of the degree of disorder of the yarn structure is performed.

To analyze the lateral deviations of the image of the yarn 3 envelope from the straight direction of yarn 3, changes in the positions of the edges H of the yarn image relative to the yarn optical sensor 6 during yarn movement, e.g. during yarn production or yarn winding, etc. are evaluated, whereby one range O of frequencies of the spectrum of the positions of the edges H of the yarn 3 during the movement of yarn and spectrum E of the positions of the edges H of the image of the yarn 3 in a determined or set at least one range 0 of frequencies is continuously monitored and evaluated. From the monitored and continuously evaluated spectrum E of the positions of the edges H of the yarn 3 image in the at least one determined range O of frequencies, the waviness K of the yarn 3 surface is determined as a function of the deviations of the yarn 3 envelope from the straight direction of yarn 3. The current lateral deviations of the image of the yarn 3 envelope from the straight direction of yarn 3 are continuously statistically evaluated and are compared with a reference value Kref of the waviness K of the yarn surface, wherein the reference value Kref of the waviness K of the yarn 3 surface is formed either as the long-term average of the values of the waviness K of the yarn 3 surface from the same unit 1 and/or as the long-term average of the values of the waviness K of the yarn 3 surface from multiple units 1 which are set to produce or process or measure the same yarn 3 and/or the reference value Kref of the waviness K of the yarn 3 surface is set according to the current production or processing or measuring parameters of the production or processing or measuring machine and a structural defect V of yarn is detected upon occurrence of the current deviations ΔK of the waviness K of the yarn 3 surface with respect to the reference value Kref of the waviness K of the yarn 3 by more than the set decision threshold.

According to one embodiment, the range of the analyzed frequencies of spectrum E of the positions of the edges H of fasciated yarn 3 is set depending on the current yarn parameters 3, especially on the type of fibrous material for yarn production 3, on the speed of yarn movement 3, on the spinning pressure in the spinning nozzle 20, on the fineness of the spun yarn 3 and depending on the current components of the spinning unit 2, especially on the type of spinning nozzle 20, etc.

To analyze disorder of the structure of fasciated yarn 3, at least one frequency domain OL for monitoring the image of wild fibers 32 on at least a portion of the yarn length 3, the domain OL being significant for monitoring the disorder in the yarn structure 3, is determined or set and the disorder of the structure of fasciated yarn 3 is monitored and evaluated in this domain OL. From the disorder in the yarn 3 structure, the yarn 3 wildness W is determined as a function of occurrence of irregularities in the structure of fasciated yarn and/or as a function of occurrence of irregularities in the surface structure of fasciated yarn 3. The yarn 3 wildness W is monitored and compared with a reference value Wref of the yarn 3 wildness W, whereby the reference value Wref of the yarn 3 wildness W is formed either as the long-term average of the values of the yarn wildness W from the same unit 1 and/or as the long-term average of the values of the yarn wildness W from multiple units 1 set to produce the same yarn and/or the reference value Wref of the yarn 3 wildness W is set according to the current production or processing or measuring parameters of the production or processing or measuring machine and a structural defect V of the yarn is detected when deviations ΔW of the current yarn wildness W from the reference value Wref of the yarn wildness W exceed the set decision threshold

It is advantageous if the domain OL for monitoring the image of wild fibers 32 on at least a portion of the fasciated yarn 3 length is set depending on the current yarn parameters 3, in particular on the type of fibrous material 11, 14 for producing yarn 3, the speed of yarn 3 movement, on the spinning pressure in the spinning nozzle 20, on the fineness of the spun yarn 3 and depending on the current components of the spinning unit 2, especially on the type of spinning nozzle 20, etc.

The analysis of lateral deviations of the image of the envelope of fasciated yarn 3 from the straight direction of yarn 3 and the analysis of the disorder in the yarn 3 structure are preferably performed concurrently to achieve more accurate results and to increase reliability of detection of the structural defects V in the yarn 3.

For the efficient use of the means at the workstation 1 of the machine, at least one average parameter of the yarn 3, especially diameter Φ of the yarn 3, is sensed by an optical sensor 6 of yarn 3 quality simultaneously with capturing the image of fasciated yarn 3 for detecting a structural defect V of yarn.

The device for detecting structural defects of fasciated yarn 3 has at least one optical sensor 6 of yarn 3 with at least one row 610 of radiation-sensitive elements 6100. The device further comprises at least one radiation source 62 and is also provided with means adapted to perform a method for optical detection of structural defects V of fasciated yarn 3, e.g., during the production of fasciated yarn 3 on the air-jet spinning machine or during the winding of the previously produced yarn 3, etc. According to a preferred embodiment, the optical yarn sensor 6 is formed by an optical sensor of yarn quality. The means adapted to perform the method for optical detecting structural defects V of fasciated yarn 3, or electronics 7 comprise a microprocessor with a memory or a gate array or a customer electronic circuit of the ASIC type or comprise a combination of at least two of these elements.

The computer program (product) for implementing the present invention includes instructions for the device for the optical detection of structural defects of fasciated yarn 3 performing the steps of the method for the optical detection of structural defects of fasciated yarn 3. This computer program is preferably stored in a computer readable medium.

The following description focuses on the description of examples of an arrangement of an air-jet spinning machine for producing fasciated yarn which comprises a core 30 formed by parallel fibers, the core 30 being wrapped by a wrapping layer of fibers comprising wrapper fibers 31 and wild fibers 32, and on the description of examples of image acquisition and image processing of yarn 3 and the evaluation of the yarn 3 image for the purposes of the optical detection of structural defects V of yarn 3 during the production of fasciated yarn 3 on the air-jet spinning machine. Adequately, this description can also be used for winding machines and other textile machines processing fasciated yarn 3 or otherwise handling fasciated yarn 3, e.g., specialized textile machines or measuring machines, etc.

The air-jet spinning machine comprises at least one row of identical spinning stations 1 arranged next to each other. Each spinning station 1 comprises a container of fibrous material for producing fasciated yarn 3. The container is typically formed by a sliver can 10 in which a sliver 11 of fibers is stored. The spinning station 1 further comprises a drafting device 13 to which the sliver 11 is fed from the can 10 and which reduces the sliver 11 into a formation 14 suitable to be fed to a spinning nozzle 20 which is arranged in a spinning unit 2 which is part of the spinning station 1.

The spinning unit 2 is adapted to convert the fibrous formation 14 in the spinning nozzle 20 into yarn 3 which is withdrawn from the spinning unit 2 by a draw-off mechanism 4 arranged at the spinning station 1 in the direction of the fibrous material movement, here already in the form of yarn 3, downstream of the spinning unit 2. The spinning station 1 further comprises a traversing and ding device 5 of yarn 3 on a bobbin 50 which is arranged at the spinning station 1 in the direction of the fibrous material movement downstream of the draw-off mechanism 4 of yarn 3 and which is adapted to traverse the yarn 3 across the width of the rotating bobbin 50 and to wind the yarn 3 onto the rotating bobbin 50.

At least one optical sensor 6 of yarn 3 is arranged between the outlet 21 of yarn 3 from the spinning unit 2 and the traversing and winding device 5 of yarn 3 onto the bobbin 50. The optical sensor 6 of yarn 3 is adapted to perform the optical sensing of the yarn 3 during the production of yarn 3 at the spinning station 1 of the air-jet spinning machine, i.e., to capture a yarn 3 image during the production of yarn 3. The optical yarn 3 sensor 6 is connected to electronics 7 which is equipped with software. The optical sensor 6 of yarn 3 and the electronics 7 are adapted to capture yarn images and process them according to the invention, i.e., to obtain and process information on the visual character of fasciated yarn 3, or, more specifically, data on the visual character of the surface structure of fasciated yarn provided by the optical sensor 6, whether in the form of a yarn image or in the form of an output signal corresponding to some of the monitored parameters, both for the detection of structural defects of yarn 3 during the yarn production at the spinning station 1 of the air-jet spinning machine.

The optical sensor 6 typically comprises at least one radiation source 60 which is arranged in a suitable position relative to the yarn 3, or in a suitable position relative to the path of the yarn 3 passing through the optical sensor 6. The optical sensor 6 further comprises an optical sensing element 61, relative to which the radiation source 60 is also suitably arranged.

In an exemplary embodiment shown in Figs. 1 to 2a, the radiation source 60 is arranged opposite the sensing element 61, whereby between the radiation source 60 and the sensing element 61 is arranged a measuring slot 62 for the passage of the yarn 3 during the sensing of the yarn 3 during its production. Thus, the yarn 3 passes through the measuring slot 62 as it is sensed at the spinning station 1during the yarn production.

In another exemplary embodiment, there is another suitable mutual arrangement of the radiation source 60, the sensing element 61 and the yarn 3 path, i.e., the position of the sensed yarn 3 during the production of yarn 3, including, e.g., an embodiment illustrated in Fig. 2b, wherein there is an arrangement for sensing the yarn 3 by the radiation emitted by the radiation source 60 and reflected from the yarn 3 which is located in the measuring space in front of the sensing element 61, the radiation source 60 being located on the same side of the yarn 3 as the sensing element 61, or, in other words, the radiation source 60 here irradiates the yarn 3 from the same direction from which the sensing element 61 is directed at the yarn.

In an unillustrated exemplary embodiment, the optical sensor 6 comprises at least a pair of radiation sources 60, whereby one radiation source 60 is arranged opposite the sensing element 61 and the measuring slot 62 is arranged between this radiation source 60 and the sensing element 61 and the other radiation source 60 is arranged on the same side of the yarn 3 as the sensing element 61, i.e., the radiation source 60 here irradiates the yarn 3 from the same direction from which the sensing element 61 is directed at the yarn. Therefore, this is in principle a combination of the radiation source 60 of the embodiment shown in Fig. 2a and of the radiation source of the embodiment shown in Fig. 2b. This combination of radiation sources 60 is advantageous because the radiation source 60 arranged opposite the sensing element 61, see Fig. 2a, serves to create the contour of the yarn 3 on the sensing element 61 and the radiation source 60, arranged codirectional with the sensing element 61, see Fig. 2b, serves to sense the radiation reflected from the yarn 3 and incident on the sensing element 61. The two radiation sources 60 mentioned above are in this case mutually synchronized so that the radiation emitted by them rapidly alternates, consequently one sensing element 61 is able to alternately sense not only the contours of the yarn 3, i.e. the edges H, their lateral displacements, occurrence and parameters of irregularities in the yarn 3 structure on the projection of the yarn 3 surface, such as wrapper fibers 31, wild fibers 32, free ends 320 of the wild fibers, slackened portions 321 of the wild fibers 32, short slackened portions LN of the yarn 3 core 30, protruding ends 310 of the wrapper fibers 31, protruding ends 300 of the fibers of the yarn 3 core 30, etc., see Fig. 8a, but it is also able to sense the visual appearance of the yarn 3 structure from the side facing the sensing element 61, i.e. the wrapping of the yarn 3 core 30 by the wrapper fibers 31 and by the wild fibers 32, as well as the wrapper fibers 31 as such, the wild fibers 32 as such, the free ends 320 of the wild fibers 32, the slackened portions 321 of the wild fibers 32, the short slackened portions LN of the yarn 3 core 30, the protruding ends 310 of the wrapper fibers 31, the protruding ends 300 of the fibers of the yarn 3 core 30, etc., see Fig. 8b. In order to process the yarn image and the obtained visual data on the structure of fasciated yarn 3 produced, these results of image acquisition of fasciated yarn 3 can then either be processed separately, see Figs. 8a and Fig. 8b, or it may be advantageous to combine them, see Fig. 8c, and process them together.

The radiation source 60 is formed by a suitable radiation emitter, e. g. point or multipoint or planar, etc., which is optionally preceded by suitable optics for optimizing the radiation direction to improve the process of sensing the yarn 3. For example, in the embodiment of Fig. 2a, in front of the point radiation emitter is arranged collimating optics 600 for parallelizing light rays from the radiation source 60 through the measuring slot 62 onto the sensing element 61, etc.

The sensing element 61 comprises at least one row 610 of radiation-sensitive elements 6100 (pixels), arranged next to each other, whereby the row 610 of radiation-sensitive elements 6100 is arranged with its length L substantially transversely to the yarn 3, the row 610 of radiation-sensitive elements 6100, in the embodiments shown, is arranged with its length L substantially transversely to the direction P of the yarn 3 movement during the yarn 3 production, i.e. transversely to the yarn 3 path during the production of yarn 3.

In an unillustrated exemplary embodiment, the yarn sensor 6 comprises at least two sensing elements 61, each having at least one row 610 of radiation-sensitive elements 6100, arranged next to each other, whereby the at least two sensing elements 61 are spatially oriented relative to each other and also to the yarn path 3, i.e. relative to each other and to the sensed yarn 3, at the spinning station 1 of the air-jet spinning machine, which enables to capture a multidimensional image of the yarn 3 and to perform multidimensional processing of the results of the images of the yarn 3 captured in this manner, which contributes especially to increasing the accuracy of determining occurrence of places or sections with a structural defect V of yarn 3.

In another unillustrated exemplary embodiment, a physically different yarn 3 sensor is assigned to the optical yarn sensor 6, e.g., a yarn 3 tension sensor, a capacitive yarn sensor, a yarn vibration sensor, a sound yarn sensor, etc., which makes it possible to combine appropriately and in accordance with current needs the optical principle of sensing structural defects of yarn 3 according to the present invention with physically different sensing of yarn 3.

Preferably, the optical yarn 3 sensor 6 consists of an optical sensor of yarn 3 quality, which can supply comprehensive and detailed data on the visual appearance of the sensed yarn 3, i.e., it can capture a yarn image in the required quality and at the required speed.

The electronics 7 with software comprises hardware elements which provide a computing performance and logic operation to perform software operations for processing the output yarn 3 image captured by the optical sensor 6 and for detecting structural defects V of yarn 3 during the yarn 3 production at the spinning station 1 of the air-jet spinning machine.

It is advantageous if the electronics 7 comprises a microprocessor with a memory or a gate array or a customer electronic circuit of the ASIC type or a suitable combination of at least two of these elements, i.e., a microprocessor, a gate array or a customer electronic circuit of the ASIC type, which thus constitute means adapted to perform software operations to process the output yarn 3 images captured by the optical sensor 6 and to detect structural defects V of yarn 3 during the yarn production at the spinning station 1 of the air-jet spinning machine.

The software contained in the computer readable medium, which is part of the electronics 7, is provided with program blocks or with instructions which are adapted to perform software operations to process the yarn 3 image captured by the optical sensor 6 and to detect structural defects V of yarn and which cause the entire device for detecting structural defects V of yarn 3 according to the present invention, which comprises at least one yarn 3 optical sensor 6 with at least one row 610 of radiation-sensitive elements 6100 arranged next to each other, the optical yarn sensor 6 being connected to the electronics 7 with software, to behave according to the required method, i.e. to perform the steps of the method according to the invention.

The computer readable medium for the present invention comprises a computer program and software according to the above paragraph.

The fasciated yarn 3 produced, e.g., at the spinning station 1 of the air-jet spinning machine, comprises a core 30 formed by a bundle of substantially parallel fibers, whereby the yarn 3 core 30 is wrapped by a wrapping layer of fibers which comprises wrapper fibers 31 and wild fibers 32. The wrapper fibers 31 are wrapped around the yarn 3 core 30 in helices with a relatively regular pitch R of the helix. The wild fibers 32, too, are wrapped around the yarn 3 core 30 in helices, but these helices have a variable irregular pitch along the length of yarn 3, as shown in Fig. 3 by means of signs R1, R2, R3, or the wild fibers 32 have an unfinished wrapping of the yarn 3 core 30 and thus form untwisted (free) ends 320 which protrude from the yarn 3 core 30. Alternatively, the wild fibers 3 have slackened portions 321 forming tufts or loops on the yarn surface. In addition, fasciated yarn exhibits short slackened portions LN of the yarn 3 core, protruding ends 310 of the wrapper fibers 31, protruding ends 300 of the core fibers 30 of yarn 3, etc., and protruding or free ends 300, 310 and 320.

An example of application of the invention in the production of fasciated yarn 3 at the spinning station 1 of the air-jet spinning machine consists in that an image of at least a part of the length of the moving fasciated yarn 3 is captured by the optical yarn 3 sensor 6, thereby obtaining data D1 on the appearance (image) of the yarn 3, i.e., data on the visual appearance of the yarn 3, or, more specifically, data on the visual structure of the yarn 3. From these data D1, descriptive data D2 are subsequently extracted, the data D2 describing how the yarn 3 core 30 is wrapped by the wrapper fibers 31 and/or by the wild fibers 32, in other words, describing what the visual yarn 3 structure is. From the extracted descriptive data D2, the wrapping S of the yarn 3 core 30 by the wrapper fibers 31 and/or the wild fibers 32 is determined. The wrapping S of the yarn 3 core 30 by the wrapper fibers 31 and/or the wild fibers 32 is determined continuously during the production of yarn 3 at the spinning station 1 of the air-jet spinning machine, whereby the changes in the wrapping S of the yarn 3 core 30 by the wrapper fibers 31 and/or by the wild fibers 32 are detected. If a change in the wrapping S of the yarn 3 core 30 by the wrapper fibers 31 and/or the wild fibers 32 is detected, i.e., found, then the corresponding place of occurrence of a yarn 3 structural defect V is determined, as is shown, for example, in Fig. 4.

Furthermore, due to the speeds of the yarn 3 movement at the spinning station 1 of the air-jet spinning machine it appears that it is possible to determine very well the wrapping S of the yarn 3 core 30 by the wrapper fibers 31 and/or wild fibers 32 from the visual changes in the yarn 3 surface structure, i.e. the structure of the yarn 3 surface in the vicinity of the edges H of the yarn 3, from the movements of the yarn 3 edges H, etc., all this in the monitoring zone Z along the yarn 3, or in the yarn 3 envelope.

It appears that the determination of the visual changes in the yarn 3 surface structure can be performed by making analysis of the yarn 3 surface structure aimed at determining the waviness K of the yarn 3 surface and/or determining the yarn 3 wildness W from the character of the wild fibers 32.

If a deviation ΔK of the waviness K of the yarn 3 surface is detected, e.g., compared with the long-term statistical average of the waviness K of the yarn 3 surface by more than the set decision threshold, e.g. by more than 5 %, and/or if a deviation ΔH of the yarn 3 wildness W is detected, e.g., compared with the long-term statistical average of the yarn 3 wildness W , by more than the set decision threshold, e.g. by more than 5 %, then the corresponding place of occurrence of a yarn 3 structural defect V is determined, as is illustrated, for example, in Fig. 4.

The waviness K of the surface of fasciated yarn can be well determined in the frequency domain of spectrum E of the position of the edges H of the yarn 3 surface in the direction of the width of the sensing element 61 of the optical sensor 6, or in the direction of the length of at least one row 610 of the elements 6100 of the sensing element 61 of the yarn 3 optical sensor 6, which are arranged next to each other. In this frequency domain, at least one range O of frequencies is determined or set, the frequencies being significant for the monitoring of spectrum E of the position of the edges H (change ΔH in the position of the edges) of the yarn 3 surface and in at least one determined or set range O, this spectrum E of the position of the edges H of the yarn 3 surface is monitored and continuously evaluated. From the monitoring and continuous evaluation of spectrum E of the position of the edges H of the yarn 3 surface in at least one determined or set range O of frequencies, the waviness K of the yarn 3 surface is determined, which essentially describes the behavior of the pitch R of the helix of the wrapper fibers 31 on the yarn 3 core 30 in the length direction, or the direction of the movement P of the sensed yarn 3, i.e., it essentially describes the wrapping of the yarn 3 core 30 by the wrapper fibers 31.

The improved determination of the corresponding place of occurrence of a structural defect of yarn 3 is performed by the monitoring and continuous statistic evaluation of the deviation ΔK of the current waviness K of the yarn 3 surface waviness from the reference waviness Kref of the yarn 3 surface. The reference waviness Kref of the yarn 3 surface is either determined as the long-term average of the waviness K of the yarn 3 surface from the same spinning unit 2 and/or as the long-term average of the waviness K of the yarn 3 surface from a plurality of spinning stations 2 which are set to produce the same yarn 3 and/or the reference waviness Kref of the yarn 3 surface is set according to the current production parameters at the spinning station 1 of the air-jet spinning machine.

To detect a place of occurrence of a yarn structural defect V, occurrence of deviations ΔK of the currently detected waviness K of the yarn 3 surface from the set threshold, for example by more than 5 %, is detected.

According to another embodiment of the invention using the determination of the waviness K of the yarn 3 surface in the frequency domain of spectrum E of the position of the edges H of the yarn 3 surface in the direction of the width of the sensing element 61 of the optical sensor 6, the setting of at least one range O of frequencies which are important for the monitoring of spectrum E of the position of the edges H of the yarn surface (change ΔH in the position of the edges) compared with the long-term average of spectrum E of the position of the edges H of the yarn 3 surface is performed depending on the current production parameters of yarn 3, especially on the type of fibrous material 11, 14 for yarn 3 production, on the speed of yarn 3 movement, on the spinning pressure in the spinning nozzle 20, on the fineness of the spun yarn 3 and depending on the current components of the spinning unit 2, especially on the type of spinning nozzle 20.

The wrapping S of the yarn 3 core 30 can be well determined by determining the yarn 3 wildness W, which in principle evaluates the degree of disorder of the yarn 3 structure, whereby this assessment includes evaluating the wrapping of the yarn 3 core 30 by the wrapper fibers 31 and by the wild fibers 32, as well as evaluating the wrapper fibers 31 as such, the wild fibers as such, free ends 320 of the wild fibers 32, slackened portions 321 of the wild fibers 32, short slackened portions LN of the yarn 3 core 30, protruding ends 310 of the wrapper fibers 31, protruding ends 300 of the yarn 3 core 30 fibers, etc., all this in the time domain of the spectrum of the wild fibers 32, in which at least one domain OL, significant for monitoring the disorder in the yarn 3 structure, represented by the above-mentioned parameters, is determined or set in a limited area Z along the edges H of the yarn 3 core 30. In this determined or set domain OL of the spectrum of the disorder in the yarn 3 structure 3, the wrapping of the yarn 3 core 30 is monitored and evaluated, i.e., the wrapping of the yarn 3 core 30 by the wrapper fibers 31 and by the wild fibers 32, the wrapper fibers 31 as such, the wild fibers as such, the free ends 320 of the wild fibers 32, the slackened portions 321 of the wild fibers 32, the short slackened portions LN of the yarn 3 core 30, the protruding ends 310 of the wrapper fibers 31, the protruding ends 300 of the fibers of the yarn 3 core 30, etc.

The wildness W of fasciated yarn 3 is used to evaluate structural defects of yarn 3 according to the present invention by monitoring and continuously statistically evaluating deviation ΔW of the current yarn 3 wildness W from the reference value Wref of the yarn 3 wildness. The reference value Wref of the yarn 3 wildness is either the long-term average of the yarn 3 wildness W from the same spinning unit 2 and/or is the long-term average of the yarn 3 wildness W from a plurality of spinning stations 2 which are set to produce the same yarn 3 and/or the reference value Wref of the yarn 3 wildness is set according to the current production parameters at the spinning station 1 of the air-jet spinning machine.

For easy and quick evaluation, occurrence of deviations ΔW of the yarn 3 wildness W by more than the set decision threshold, e.g., by more than 5 %, is detected for the determination of occurrence of a structural defect V of yarn 3.

In order to facilitate the setting of the detection of occurrence of a structural defect V of yarn 3, according to another embodiment of the invention, the setting of the area important for determining the value W of the yarn 3 wildness is performed according to the current yarn 3 production parameters, especially according to the type of fibrous material 11, 14 for the yarn 3 production, the speed of yarn 3 movement, the spinning pressure in the spinning nozzle 20, the fineness of the spun yarn 3 and according to the current components of the spinning unit 2, especially on the type of spinning nozzle 20, etc.

So as to improve the accuracy of detecting a structural defect V of yarn 3, the waviness K of the yarn 3 surface and the yarn 3 wildness W are monitored continuously and the overall wrapping KWS of the yarn 3 core 30 by the wrapper fibers 31 and the wild fibers 32 is determined from the deviations of the waviness K of the yarn 3 surface and from the deviations of the yarn 3 wildness W.

To detect a structural defect V of yarn 3, deviations ΔKWS of the current overall wrapping KWS of the yarn 3 core 30 from the reference degree KWSref of the overall wrapping KWS of the yarn 3 core 30 are monitored and continuously statistically evaluated. The reference degree KWSref of the overall wrapping KWS of the yarn 3 core 30 is either the long-term average of the overall wrapping KWS of the yarn 3 core 30 produced at the same spinning unit 2 and/or is the long-term average of the overall wrapping KWS of the yarn 3 core 30 produced at multiple spinning stations 2 which are set to produce the same yarn 3 and/or the overall wrapping KWS of the yarn 3 core 30 is set according to the current production parameters at the spinning station 1 of the air-jet spinning machine.

In another exemplary embodiment, fasciated yarn 3 is sensed by the optical sensor 6 of yarn 3 quality which senses simultaneously at least one diametric parameter of yarn 3, in particular diameter Φ of yarn 3.

### Industrial applicability

The invention is applicable on air-jet spinning machines in the production of fasciated yarn. In addition, it is applicable on winding machines and in principle it is generally applicable also on other types of textile machines for producing and/or processing and/or analyzing or measuring fasciated yarn, where it is necessary or desirable to evaluate the fasciated yarn produced or processed not only from the point of view of diametric characteristics and in terms of hairiness, but also from the point of view of changes in the structure of the yarn produced and/or processed.

### List of references

- 1: spinning station
- 10: sliver can
- 11: sliver
- 13: drafting device
- 14: fibrous formation
- 2: spinning unit
- 20: spinning nozzle
- 21: outlet of yarn from the spinning nozzle
- 3: fasciated yarn
- 30: core of fasciated yarn
- 300: protruding end of the fasciated yarn core
- 31: wrapper fiber
- 310: protruding end of a wrapper fiber
- 32: wild fiber
- 320: free end of a wild fiber
- 321: slackened portion of a wild fiber
- 4: draw-off mechanism
- 5: winding device
- 50: bobbin
- 6: optical yarn sensor
- 60: radiation source
- 600: collimation optics
- 61: optical sensing element
- 610: row of radiation-sensitive elements arranged next to each other
- 6100: radiation-sensitive element
- 62: measuring slot
- 7: electronics
- D1: data on the visual appearance of yarn (on the yarn image)
- D2: descriptive data on how the yarn core is wrapped by the wrapper fibers and/or wild fibers, or on what the visual structure of yarn is
- E: spectrum of the positions of the edges of the yarn surface in the direction of the width of the sensing element of the optical sensor
- Φ: yarn diameter
- H: yarn edge
- ΔH: change in the positions of the yarn edges
- K: yarn surface waviness
- ΔK: deviation of the yarn surface waviness
- Kref: reference waviness of yarn surface
- KWS: overall wrapping of the yarn core by the wrapper fibers and the wild fibers
- ΔKWS: reference overall wrapping of the yarn core
- L: length of the row of radiation-sensitive elements arranged
- LN: short portion of slackening in the yarn core
- O: range of frequencies that are significant for monitoring the spectrum of edge positions (edge position changes) of the yarn surface
- OL: frequency domain which is significant for monitoring disorder in the yarn structure in the time domain of the spectrum of the wild fibers in a limited area along the edges of the yarn core
- P: direction of yarn movement during production
- R: pitch of helix of the wrapper fibers
- R1, R2, R3: pitch of helix of the wild fibers
- S: wrapping of the yarn core
- V: structural defect of yarn
- W: yarn wildness
- ΔW: deviation of the yarn wildness
- Wref: reference yarn wildness
- Z: monitoring zone along the yarn

## Claims

1. A method for detecting structural defects (V) of fasciated yarn (3) comprising a core (30) formed by parallel fibers, whereby the core (30) is bound together by a wrapping (S) layer of fibers which comprises wrapper fibers (31) and wild fibers (32), the wild fibers (32) being fibers wound around the yarn core (30) in helices having a variable pitch (R, R1, R2, R3) or a variable direction or wound around the yarn core (30) imperfectly, where the structural defects (V) of fasciated yarn (3) in a particular place or section do not significantly affect the mass and/or diametric characteristics of the fasciated yarn (3) or the parameter of yarn hairiness in these places or sections of the fasciated yarn (3), the method being **characterized in that**,
- an image of moving yarn (3) is captured by at least one optical yarn sensor (6) on at least a portion of the yarn (3) length,
- whereby the optical yarn sensor (6) comprises at least one row (610) of radiation-sensitive elements (6100) and at least one radiation source (60),
- the optical sensor (6) is connected to electronics (7) equipped with software,
- whereby the captured image of yarn (3) is processed by a digital image analysis method to obtain data on the yarn surface structure,
- from the data obtained, changes in the yarn surface structure along the yarn length are evaluated,
- these changes in the yarn surface structure are compared with pre-set criteria,
- and if these changes in the yarn surface structure exceed the pre-set criteria, the relevant place of yarn (3) is determined as a place of occurrence of the structural defect (V) of yarn.

2. The method according to claim 1, in which
- the optical sensor (6) captures an image of fasciated yarn (3) along the yarn edges (H),
- the captured image of the yarn (3) is processed using by a digital image analysis method to obtain data on the yarn structure along the yarn edges (H),
- from the data obtained, changes in the yarn structure along the yarn edges (H) are evaluated,
- these changes in the yarn structure along the yarn edges (H) are compared with pre-set criteria,
- and if these changes in the yarn structure along the yarn edges (H) exceed the pre-set criteria, the relevant place of yarn (3) is determined as the place of occurrence of the structural defect (V) of yarn (3).

3. The method according to any of claims 1 to 2, in which
- simultaneously with the capturing of the yarn (3) image for detecting the structural defect (V) of fasciated yarn (3), the optical yarn quality sensor (6) senses at least one yarn diametric parameter, in particular yarn diameter (Φ).

4. A device for detecting structural defects (V) of fasciated yarn (3), where the structural defects (V) of fasciated yarn (3) in a particular place or section do not significantly affect the mass and/or diametric characteristics of the fasciated yarn (3) or the parameter of yarn hairiness in these places or sections of the fasciated yarn (3), the device being **characterized by** having at least one optical yarn sensor (6) with at least one row (610) of radiation-sensitive elements (6100), and further having at least one radiation source (60), as well as means adapted to perform the method according to any of claims 1 to 3.

5. The device according to claim 4, wherein
- the optical yarn sensor (6) is formed by an optical sensor of yarn quality.

6. The device according to claim 4 or 5, wherein
- the means adapted to perform the method according to any of claims 1 to 3 comprise a microprocessor with a memory or a gate array or a customer electronic circuit of the ASIC type or comprise a combination of at least two of these elements.

7. A computer program (product) containing instructions causing the device according to any of claims 4 to 6 to perform the steps of the method according to any of claims 1 to 3.

8. A computer readable medium which has the computer program stored therein according to claim 7.

## Patentansprüche

1. Verfahren zum Erkennen von Strukturfehlern (V) eines gebündelten Garns (3), das einen aus parallelen Fasern gebildeten Kern (30) aufweist, wobei der Kern (30) von einer Deckschicht (S) von Fasern umhüllt ist, die Deckfasern (31) und wilde Fasern (32) aufweist, die wilden Fasern (32) solche Fasern sind, die in Schraubenlinien mit variabler Steigung (R, R1, R2, R3) oder mit variabler Richtung um den Kern (30) des Garns gewickelt sind oder die nicht vollkommen um den Kern (30) des Garns gewickelt sind, wo die Strukturfehler (V) des gebündelten Garns (3) an der relevanten Stelle oder im relevanten Teil die Gewichts- und/oder Durchmessereigenschaften des gebündelten Garns (3) oder den Haarigkeitsparameter des Garns an diesen Stellen oder in Teilen des gebündelten Garns (3) nicht signifikant beeinflussen,
das Verfahren ist **dadurch gekennzeichnet, dass**
- das Bild des sich bewegenden Garns von mindestens einem optischen Garnsensor (6) über mindestens einen Teil der Länge des Garns (3) abgetastet wird,
- wobei der optische Garnsensor (6) mindestens eine Reihe (610) strahlungsempfindlicher Elemente (6100) und mindestens eine Strahlungsquelle (60) aufweist,
- der optische Sensor (6) an eine mit Software ausgestattete Elektronik (7) angeschlossen ist,
- wobei das abzutastende Bild des Garns (3) mittels eines digitalen Bildanalyseverfahrens verarbeitet wird, um Daten über die Garnoberflächenstruktur zu erhalten,
- anhand dieser Daten die Veränderungen der Garnoberflächenstruktur entlang der Garnlänge ausgewertet werden,
- diese Veränderungen der Garnoberflächenstruktur mit voreingestellten Kriterien verglichen werden,
- und wenn diese Veränderungen der Garnoberflächenstruktur diese voreingestellten Kriterien überschreiten, wird die entsprechende Stelle des Garns (3) als Stelle des Auftretens eines Strukturfehlers (V) des Garns bestimmt.

2. Verfahren nach dem Anspruch 1, bei dem
- durch den optischen Sensor (6) das Bild des gebündelten Garns (3) entlang der Ränder (H) des Garns abgetastet wird,
- das abzutastende Bild des Garns (3) mittels eines digitalen Bildanalyseverfahrens verarbeitet wird, um Daten über die Garnstruktur entlang der Ränder (H) des Garns zu erhalten,
- anhand der erhaltenen Daten die Veränderungen der Garnstruktur entlang der Ränder (H) des Garns ausgewertet werden,
- diese Veränderungen der Garnstruktur entlang der Ränder (H) des Garns mit voreingestellten Kriterien verglichen werden,
- und wenn diese Veränderungen der Garnstruktur entlang der Ränder (H) des Garns diese voreingestellten Kriterien überschreiten, wird die entsprechende Stelle des Garns (3) als Stelle des Auftretens eines Strukturfehlers (V) des Garns bestimmt.

3. Verfahren nach einem der Ansprüche 1 bis 2, bei dem
- gleichzeitig mit dem Abtasten des Bildes des Garns (3) zum Erkennen des Strukturfehlers (V) des gebündelten Garns (3) mit dem optischen Garnqualitätssensor (6) mindestens ein Durchmesserparameter des Garns, insbesondere der Durchmesser (Φ) des Garns, abgetastet wird.

4. Einrichtung zum Erkennen von Strukturfehlern (V) eines gebündelten Garns (3), wo die Strukturfehler (V) des gebündelten Garns (3) an einer relevanten Stelle oder in einem relevanten Teil die Gewichts- und/oder Durchmessereigenschaften des gebündelten Garns (3) oder den Haarigkeitsparameter des Garns an diesen Stellen oder in den Teilen des gebündelten Garns (3) nicht signifikant beeinflussen, die Einrichtung ist **dadurch gekennzeichnet, dass** sie mindestens einen optischen Garnsensor (6) mit mindestens einer Reihe (610) strahlungsempfindlicher Elemente (6100) sowie mindestens eine Strahlungsquelle (60) sowie Mittel aufweist, die zum Ausführen des Verfahrens nach einem der Ansprüche 1 bis 3 angepasst sind.

5. Einrichtung nach dem Anspruch 4, wo
- der optische Garnsensor (6) durch einen optischen Garnqualitätssensor gebildet ist.

6. Einrichtung nach dem Anspruch 4 oder 5, wo
- die Mittel, die zur Ausführung des Verfahrens nach einem der Ansprüche 1 bis 3 angepasst sind, einen Mikroprozessor mit einem Speicher oder ein Gate-Array oder eine kundenspezifische elektronische Schaltung Typ ASIC oder eine Kombination aus mindestens zwei dieser Elemente aufweisen.

7. Computerprogramm (Produkt), das die Anweisungen aufweist, die verursachen, dass die Einrichtung nach einem der Ansprüche 4 bis 6 die Schritte des Verfahrens nach einem der Ansprüche 1 bis 3 ausführt.

8. Durch Rechner lesbares Medium, das ein Computerprogramm nach Anspruch 7 gespeichert aufweist.

## Revendications

1. Procédé de détection de défauts structurels (V) d'un fil en faisceau (3) qui comprend un noyau (30) formé de fibres parallèles, tandis que le noyau (30) est enveloppé d'une couche (S) de fibres qui comprend des fibres enveloppantes (31) et des fibres sauvages (32), les fibres sauvages (32) étant des fibres enroulées autour du noyau (30) du fil en hélice avec un pas variable (R, R1, R2, R3) ou avec une direction variable, ou étant enroulées de manière imparfaite autour du noyau (30) du fil, où les défauts structurels (V) du fil en faisceau (3) à l'endroit ou dans la partie concernée n'influencent pas de manière significative les caractéristiques de poids et/ou de diamètre du fil en faisceau (3), ni le paramètre de pilosité du fil à ces endroits ou dans ces parties du fil en faisceau (3),
le procédé étant **caractérisé en ce que**
- l'image du fil en mouvement est captée par au moins un capteur optique (6) du fil sur au moins une partie de la longueur du fil (3),
- tandis que le capteur optique (6) du fil comprend au moins une rangée (610) d'éléments sensibles au rayonnement (6100) et au moins une source de rayonnement (60),
- le capteur optique (6) est relié à un système électronique (7) équipé d'un logiciel ;
- tandis que l'image détectée du fil (3) est traitée par une méthode d'analyse d'image numérique afin d'obtenir des données sur la structure de la surface du fil,
- à partir de ces données obtenues, les modifications de la structure de la surface du fil sont évaluées sur la longueur du fil,
- ces modifications de la structure de la surface du fil sont comparées à des critères prédéfinis,
- et si ces modifications de la structure de la surface du fil dépassent ces critères prédéfinis, l'emplacement correspondant du fil (3) est déterminé comme emplacement d'apparition d'un défaut structurel (V) du fil.

2. Procédé selon la revendication 1, lors duquel
- un capteur optique (6) détecte l'image du fil en faisceau (3) le long des bords latéraux (H) du fil,
- l'image capturée du fil (3) est traitée par une méthode d'analyse d'image numérique afin d'obtenir des données sur la structure du fil le long des bords latéraux (H) du fil,
- à partir de ces données obtenues sont évaluées les modifications de la structure du fil le long des bords latéraux (H) du fil,
- ces modifications de la structure du fil le long des bords latéraux (H) du fil sont comparées à des critères prédéfinis,
- et si ces modifications de la structure du fil le long des bords latéraux (H) du fil dépassent ces critères prédéfinis, l'emplacement correspondant du fil (3) est déterminé comme emplacement d'apparition d'un défaut structurel (V) du fil.

3. Procédé selon l'une des revendications de 1 à 2, lors duquel
- simultanément à l'acquisition de l'image du fil (3) pour la détection d'un défaut structurel (V) du fil en faisceau (3), au moins un paramètre moyen du fil, en particulier le diamètre (Φ) du fil, est mesuré par un capteur optique (6) de qualité du fil.

4. Dispositif de détection des défauts structurels (V) d'un fil en faisceau (3), où les défauts structurels (V) du fil en faisceau (3) à l'endroit ou dans la partie concernée n'influencent pas de manière significative les caractéristiques de poids et/ou de diamètre du fil en faisceau (3), ni le paramètre de pilosité du fil à ces endroits ou dans ces parties du fil en faisceau (3), le dispositif étant **caractérisé en ce qu'**il comporte au moins un capteur optique (6) de fil avec au moins une rangée (610) d'éléments sensibles au rayonnement (6100), et au moins une source (60) de rayonnement ainsi que des moyens adaptés pour mettre en œuvre le procédé selon l'une des revendications de 1 à 3.

5. Dispositif selon la revendication 4, où
- le capteur optique (6) de fil est constitué d'un capteur optique de qualité de fil.

6. Dispositif selon la revendication 4 ou 5, où
- les moyens adaptés pour mettre en œuvre le procédé selon l'une des revendications de 1 à 3 comprennent un microprocesseur avec mémoire ou un réseau de portes ou un circuit électronique personnalisé de type ASIC, ou une combinaison d'au moins deux de ces éléments.

7. Programme informatique (produit) contenant des instructions amenant le dispositif selon l'une des revendications de 4 à 6 à exécuter les étapes du procédé selon l'une des revendications de 1 à 3.

8. Support lisible par ordinateur sur lequel est enregistré le programme informatique selon la revendication 7.
